# EUROPEAN PATENT SPECIFICATION

(11) **EP 2 576 787 B1**
(45) Date of publication and mention of the grant of the patent: **03.05.2017**
(21) Application number: 10852344.0
(22) Date of filing: 31.05.2010
(51) Int. Cl.: C12N 15/12, C12N 15/11, C12Q 1/68

(54) **SNPS ASSOCIATED WITH POLYCYSTIC OVARY SYNDROME, CHIPS COMPRISING THE SAME AND USE THEREOF**
MIT POLYZYSTISCHEM OVARIALSYNDROM ASSOZIIERTE SNPS, CHIPS DAMIT UND IHRE VERWENDUNG
POLYMORPHISMES MONONUCLÉOTIDIQUES ASSOCIÉS AU SYNDROME DES OVAIRES POLYKYSTIQUES, PUCES EN CONTENANT ET LEUR UTILISATION

(43) Date of publication of application: 10.04.2013
(73) Proprietor: Shandong University, Jinan, Shandong 250100 (CN); Shandong Shanda Hospital for Reproductive Medicine Co., Ltd., Shandong 250021 (CN); Shanghai Jiao Tong University, Shanghai 200030 (CN)
(72) Inventor: CHEN, Zijiang, Jinan Shandong 250021 (CN); HE, Lin, Shanghai 200030 (CN); SHI, Yongyong, Shanghai 200030 (CN); MA, Jinlong, Jinan Shandong 250021 (CN); ZHAO, Yueran, Jinan Shandong 250021 (CN); ZHAO, Han, Jinan Shandong 250021 (CN); SHI, Yuhua, Jinan Shandong 250021 (CN); GENG, Ling, Jinan Shandong 250021 (CN); YOU, Li, Jinan Shandong 250021 (CN)
(74) Representative: Hautier IP
(86) International application number: PCT/CN2010/073387
(87) International publication number: WO 2011/150545

(56) References cited:
- EP-A1- 1 749 891
- WO-A1-2006/125513
- WO-A1-2009/105655
- KR-A- 20040 074 800
- KR-A- 20040 074 800
- K. G. EWENS ET AL: "Family-Based Analysis of Candidate Genes for Polycystic Ovary Syndrome", JOURNAL OF CLINICAL ENDOCRINOLOGY & METABOLISM, vol. 95, no. 5, 1 May 2010 (2010-05-01), pages 2306-2315, XP055054100, ISSN: 0021-972X, DOI: 10.1210/jc.2009-2703
- DATABASE dbSNP [Online] NCBI; 16 March 2004 (2004-03-16), XP002692629, retrieved from NCBI Database accession no. rs11891936
- CHENG HU ET AL: "PPARG, KCNJ11, CDKAL1, CDKN2A-CDKN2B, IDE-KIF11-HHEX, IGF2BP2 and SLC30A8 Are Associated with Type 2 Diabetes in a Chinese Population", PLOS ONE, vol. 4, no. 10, 1 January 2009 (2009-01-01), page e7643, XP055054233, ISSN: 1932-6203, DOI: 10.1371/journal.pone.0007643
- DATABASE GENBANK 11 April 2010 'Homo sapiens thyroid adenoma associated (THADA), transcript variant 3, mRNA.', XP008162782 Database accession no. NM_001083953
- DATABASE GENBANK 11 April 2010 'Homo sapiens luteinizing hormone/choriogonadotropin receptor (LHCGR), mRNA.', XP008162781 Database accession no. NM_000233
- DATABASE GENBANK 05 March 2010 'Homo sapiens DENN/MADD domain containing 1A (DENND1A), transcript variant 2, mRNA.', XP008162780 Database accession no. NM_024820
- JIN, JIA-LI ET AL.: 'Association between CYP19 gene SNP rs2414096 polymorphism and polycystic ovary syndrome in Chinese women.' BMC MEDICAL GENETICS vol. 10, no. 139, 16 December 2009, XP021066782
- AFFYMETRIX: "Data Sheet Affymetrix(R) Genome-Wide Human SNP Array 6.0", INTERNET CITATION, 2007, pages 1-4, XP002525407, Retrieved from the Internet: URL:http://www.affymetrix.com/support/tech nical/datasheets/genomewide_snp6_datasheet .pdf [retrieved on 2009-04-10]

### SEQUENCE LISTING

<110> Shandong University
   Shandong Shanda Hospital for Reproductive Medicine Co., Ltd.
   Shanghai Jiao-tong University
<120> SNPs associated with Polycystic Ovary Syndrome, chips comprising the same and the use thereof
<130> DP1P100419ZL/CNGZD/LY
<160> 56
<170> PatentIn version 3.5
<210> 1
   <211> 52
   <212> DNA
   <213> Artificial
<400> 1
   gtcaatgtcc tgttatgcaa tttctccgtt aaaaaaatta tatttgtaga ga 52
<210> 2
   <211> 52
   <212> DNA
   <213> Artificial
<400> 2
   gtcaatgtcc tgttatgcaa tttctctgtt aaaaaaatta tatttgtaga ga 52
<210> 3
   <211> 52
   <212> DNA
   <213> Artificial
<400> 3
   aaaattacac tcacatatga aaagctatat ctgtggactt gaaataacca gc 52
<210> 4
   <211> 52
   <212> DNA
   <213> Artificial
<400> 4
   aaaattacac tcacatatga aaagctgtat ctgtggactt gaaataacca gc 52
<210> 5
   <211> 52
   <212> DNA
   <213> Artificial
<400> 5
   tccctggggg aggtgtgacg gcagagctgc atttttatgg tatgccccaa ca 52
<210> 6
   <211> 52
   <212> DNA
   <213> Artificial
<400> 6
   tccctggggg aggtgtgacg gcagagttgc atttttatgg tatgccccaa ca 52
<210> 7
   <211> 52
   <212> DNA
   <213> Artificial
<400> 7
   ctgttgtaaa gcaaaataga atcctaaacc agaacttctg cagttagcca ca 52
<210> 8
   <211> 52
   <212> DNA
   <213> Artificial
<400> 8
   ctgttgtaaa gcaaaataga atcctacacc agaacttctg cagttagcca ca 52
<210> 9
   <211> 52
   <212> DNA
   <213> Artificial
<400> 9
   aaacttttac aaccagaatt aatgttccct tgtgctcttt taaaaaatca aa 52
<210> 10
   <211> 52
   <212> DNA
   <213> Artificial
<400> 10
   aaacttttac aaccagaatt aatgtttcct tgtgctcttt taaaaaatca aa 52
<210> 11
   <211> 52
   <212> DNA
   <213> Artificial
<400> 11
   tgctgtgcaa agttagaaga tgaaacaaaa ctgattacat acacctatac cc 52
<210> 12
   <211> 52
   <212> DNA
   <213> Artificial
<400> 12
   tgctgtgcaa agttagaaga tgaaaccaaa ctgattacat acacctatac cc 52
<210> 13
   <211> 52
   <212> DNA
   <213> Artificial
<400> 13
   agatgaaaca aaactgatta catacaccta taccctgcca ctaattaaaa at 52
<210> 14
   <211> 52
   <212> DNA
   <213> Artificial
<400> 14
   agatgaaaca aaactgatta catacatcta taccctgcca ctaattaaaa at 52
<210> 15
   <211> 52
   <212> DNA
   <213> Artificial
<400> 15
   tctttgttca gaagcacggt acattactat acagctgaag ccctctagca tt 52
<210> 16
   <211> 52
   <212> DNA
   <213> Artificial
<400> 16
   tctttgttca gaagcacggt acattattat acagctgaag ccctctagca tt 52
<210> 17
   <211> 52
   <212> DNA
   <213> Artificial
<400> 17
   gatcctccct atataaggcc taaaacacca ccattagagt tttactgctt ta 52
<210> 18
   <211> 52
   <212> DNA
   <213> Artificial
<400> 18
   gatcctccct atataaggcc taaaacgcca ccattagagt tttactgctt ta 52
<210> 19
   <211> 52
   <212> DNA
   <213> Artificial
<400> 19
   aggtatccac acacacccat ttcttacaca cacatcccat atcattctcg at 52
<210> 20
   <211> 52
   <212> DNA
   <213> Artificial
<400> 20
   aggtatccac acacacccat ttcttataca cacatcccat atcattctcg at 52
<210> 21
   <211> 52
   <212> DNA
   <213> Artificial
<400> 21
   attcctgctg gtcttggtta gtaccactca ataaaatgtt aggacccggg ct 52
<210> 22
   <211> 52
   <212> DNA
   <213> Artificial
<400> 22
   attcctgctg gtcttggtta gtaccattca ataaaatgtt aggacccggg ct 52
<210> 23
   <211> 52
   <212> DNA
   <213> Artificial
<400> 23
   aatttttttt tggtgattta gctacccaag aagaaaagaa gctggaatta ta 52
<210> 24
   <211> 52
   <212> DNA
   <213> Artificial
<400> 24
   aatttttttt tggtgattta gctacctaag aagaaaagaa gctggaatta ta 52
<210> 25
   <211> 52
   <212> DNA
   <213> Artificial
<400> 25
   aaatacataa gatttgggtg ctatgtaaaa agtgtcaagg caaaataatg tt 52
<210> 26
   <211> 52
   <212> DNA
   <213> Artificial
<400> 26
   aaatacataa gatttgggtg ctatgtgaaa agtgtcaagg caaaataatg tt 52
<210> 27
   <211> 52
   <212> DNA
   <213> Artificial
<400> 27
   ctcatttcta ggcagaactg agtgtccttc cctaaactgc ctgtatccat ta 52
<210> 28
   <211> 52
   <212> DNA
   <213> Artificial
<400> 28
   ctcatttcta ggcagaactg agtgtctttc cctaaactgc ctgtatccat ta 52
<210> 29
   <211> 52
   <212> DNA
   <213> Artificial
<400> 29
   tgatgatgtg atgcaataca agtctcagaa tttgttggtg agagtgtaat tt 52
<210> 30
   <211> 52
   <212> DNA
   <213> Artificial
<400> 30
   tgatgatgtg atgcaataca agtctcggaa tttgttggtg agagtgtaat tt 52
<210> 31
   <211> 52
   <212> DNA
   <213> Artificial
<400> 31
   tctttttcat ggctgtttct accatcctgg aaataataat ttttaactct ct 52
<210> 32
   <211> 52
   <212> DNA
   <213> Artificial
<400> 32
   tctttttcat ggctgtttct accatcttgg aaataataat ttttaactct ct 52
<210> 33
   <211> 52
   <212> DNA
   <213> Artificial
<400> 33
   tatatgtact tattcaacat aaatccactg tttagaaaaa agtattatag ct 52
<210> 34
   <211> 52
   <212> DNA
   <213> Artificial
<400> 34
   tatatgtact tattcaacat aaatcctctg tttagaaaaa agtattatag ct 52
<210> 35
   <211> 52
   <212> DNA
   <213> Artificial
<400> 35
   taccttgtaa aaaataatcc agaaagcagt tcaagatcag cctaggcaat at 52
<210> 36
   <211> 52
   <212> DNA
   <213> Artificial
<400> 36
   taccttgtaa aaaataatcc agaaaggagt tcaagatcag cctaggcaat at 52
<210> 37
   <211> 52
   <212> DNA
   <213> Artificial
<400> 37
   tgttcagtat tatcaagctg tatatacgtt tcgacatttc atatacatga tc 52
<210> 38
   <211> 52
   <212> DNA
   <213> Artificial
<400> 38
   tgttcagtat tatcaagctg tatatatgtt tcgacatttc atatacatga tc 52
<210> 39
   <211> 52
   <212> DNA
   <213> Artificial
<400> 39
   aaacacaaac aatgagatgc tattgtcttc caatcagcct agcaaaacca ga 52
<210> 40
   <211> 52
   <212> DNA
   <213> Artificial
<400> 40
   aaacacaaac aatgagatgc tattgttttc caatcagcct agcaaaacca ga 52
<210> 41
   <211> 53
   <212> DNA
   <213> Artificial
<400> 41
   taactgcaac aactcagtgt ggataccatc atcatgtgaa agtcacccat gac 53
<210> 42
   <211> 53
   <212> DNA
   <213> Artificial
<400> 42
   taactgcaac aactcagtgt ggatactatc atcatgtgaa agtcacccat gac 53
<210> 43
   <211> 52
   <212> DNA
   <213> Artificial
<400> 43
   tgctctggca gaagaggcac atgttgaaca aatggctgca ttatggtgag at 52
<210> 44
   <211> 52
   <212> DNA
   <213> Artificial
<400> 44
   tgctctggca gaagaggcac atgttggaca aatggctgca ttatggtgag at 52
<210> 45
   <211> 52
   <212> DNA
   <213> Artificial
<400> 45
   acacatcttc tcccctatta tactcaacca gcaagcattc ccacctttaa gc 52
<210> 46
   <211> 52
   <212> DNA
   <213> Artificial
<400> 46
   acacatcttc tcccctatta tactcagcca gcaagcattc ccacctttaa gc 52
<210> 47
   <211> 52
   <212> DNA
   <213> Artificial
<400> 47
   cacctctgca aacgatgaga ccagatcaat gtttggagaa gtaaataagg gc 52
<210> 48
   <211> 52
   <212> DNA
   <213> Artificial
<400> 48
   cacctctgca aacgatgaga ccagattaat gtttggagaa gtaaataagg gc 52
<210> 49
   <211> 52
   <212> DNA
   <213> Artificial
<400> 49
   ggcagagatt ctggggactg gaaagaacta gttatgatca aggaaccaaa ag 52
<210> 50
   <211> 52
   <212> DNA
   <213> Artificial
<400> 50
   ggcagagatt ctggggactg gaaagagcta gttatgatca aggaaccaaa ag 52
<210> 51
   <211> 52
   <212> DNA
   <213> Artificial
<400> 51
   tttattttct atagcaggtt tattgacact ttttttctag taaagtttga aa 52
<210> 52
   <211> 52
   <212> DNA
   <213> Artificial
<400> 52
   tttattttct atagcaggtt tattgatact ttttttctag taaagtttga aa 52
<210> 53
   <211> 52
   <212> DNA
   <213> Artificial
<400> 53
   tgctgaaaaa aatgattgga tgatagatcg gattaagaag ggaagaaata gc 52
<210> 54
   <211> 52
   <212> DNA
   <213> Artificial
<400> 54
   tgctgaaaaa aatgattgga tgatagttcg gattaagaag ggaagaaata gc 52
<210> 55
   <211> 61
   <212> DNA
   <213> Artificial
<400> 55
   catcacaatt agcctgagtt atgcaacata gttttgttcc tttttagtat atagttggta 60
   t 61
<210> 56
   <211> 61
   <212> DNA
   <213> Artificial
<400> 56
   catcacaatt agcctgagtt atgcaacata tttttgttcc tttttagtat atagttggta 60
   t 61

## Description

### Technical field

This invention relates to SNPs associated with Polycystic Ovary Syndrome, chips comprising the same and the use thereof.

### Background

Polycystic ovary syndrome is a clinical condition characterized by presence of two or more of these features: chronic oligo-ovulation or anovulation, androgen excess and polycystic ovaries.¹ As the most common cause of anovulatory infertility, PCOS affects 6-8% childbearing-aged women.^{2,3} Additionally, PCOS is associated with important endocrine-metabolic derangements and a broad range of adverse sequelae, including dyslipidemia, atherosclerosis, insulin resistance and type 2 diabetes.⁴⁻⁶ Insulin resistance is present in perhaps 50% of women with PCOS.⁷ Among women with impaired glucose tolerance (IGT) and diabetes mellitus, about 20% were recognized at younger age to have PCOS.⁸⁻¹⁰

The pathogeneses of PCOS is not fully understood. Heritable tendencies have long been recognized, but complex interactions exist between genetic and environmental factors. Association studies have been conducted on at least 70 candidate genes, principally related to reproductive hormones, insulin resistance, and chronic inflammation, e.g., follicle stimulating hormone receptor(*FSHR*), cytochrome P450, family 11A (*CYP11A*), insulin receptor (*INSR*) and interleukin 6 (*IL-6*) ¹¹⁻¹⁵; however, none correlates consistently with PCOS.16 For example, EP1749891-A1- discloses a method and device for the in vitro detection of polycystic ovary syndrome (PCOS) and pathologies involving cardiovascular risk. An in vitro assay method and assay device (kit) for the diagnosis of the presence or the predisposition to suffer from PCOS and/or cardiovascular risk factors including: general obesity, abdominal obesity, hypertension, glucose intolerance, diabetes, hyperinsulinemia, general hypercholesterolemia, hypercholesterolemia with high LDL-cholesterol levels, low HDL-cholesterol levels and hypertriglyceridemia, as well as the grouping of some of these cardiovascular risk factors known as metabolic syndrome. The method and the kit are characterized in that they are based on the detection of at least one genotype or haplotype of a polymorphism in the CAPN5 gene selected from: Nt g.86 A>G Nt g.344 G>A, Nt c.1320 C>T and Nt c. 1469 G>A or combinations thereof.
KR20040074800-A- discloses a composition for diagnosis of polycystic ovary syndrome comprising primers detecting single nucleotide polymorphism at 677th nucleotide of 5,10-methylenetetrahydrofolate reductase gene is provided, thereby improving accuracy of diagnosis of polycystic ovary syndrome. The single nucleotide polymorphism at 677th nucleotide of MTHFR gene is selected from cytosine/cytosine(C/C), cytosine/thymine(C/T) and thymine/thymine(T/T). K. G EWENS ET AL: "Family-Based Analysis of Candidate Genes for Polycystic Ovary Syndrome", JOURNAL OF CLINICAL ENDOCRINOLOGY & METABOLISM, vol. 95, no. 5, 1 May 2010 (2010-05-01), pages 2306-2315, XP055054100, ISSN: 0021-972X, DOI: 10.1210/jc.2009-2703 found that a polymorphic variant, D19S884, in FBN3 is associated with risk of PCOS.POMC is also a candidate gene of interest.

Array based whole genome single-nucleotide polymorphisms (SNPs) association analyses may instead provide a comprehensive, unbiased approach to screen large scale patients to detect susceptibility genes for the diseases. Here a genome-wide case-control association study was carried out to identify genetic markers for PCOS in a Han Chinese population.

### Summary of Invention

We identified three susceptibility loci - 2pl6.3, 2p21and 9q33.3 for Han Chinese PCOS. In this large scale GWA study, the genetic variants most significantly associated with PCOS were further interrogated in additional independent replications. Of the 29 variants, 28 were well replicated in Northern Han Chinese PCOS subjects and 20 were replicated in Central and Southern Han Chinese. Despite the existence of genetic differentiation between Northern and Southern Han Chinese¹⁷, the risk alleles were considerably prevalent among PCOS women in China.

All PCOS implicated variants are located in one of three genes: *LHCGR* (rs13405728), *THADA* (rs12468394) and *DENNDIA* (ras 10986105), respectively. *LHCGR* encodes a G-protein coupled receptor for luteinizing hormone (*LH*) and choriogonadotropin (*HCG*). In the ovary, *LHCGR* is expressed in granulosa cells at the later stages of preovulatory follicles. Importantly, the induction of *LHCGR* during granulosa cell differentiation allows the preovulatory follicle to respond to the mid-cycle surge of LH, resulting in ovulation and release of the mature oocyte.²² In females, inactivating (or loss-of-function) mutations of *LHCGR* are associated with increased LH, enlarged ovaries, oligomenorrhea, resistance to LH or HCG and infertility;^{23,24} whereas activating mutations in affected women produce hyperandrogenism but no other effect on reproductive abnormalities.²⁵

*THADA* was initially identified in thyroid adenomas by chromosomal rearrangements resulting in disruption of *THADA* and fusion to an intron of peroxisome proliferator-activated receptor gamma (*PPARG*).²⁶ Recently, *THADA* (rs7578597) was detected as a susceptibility gene for T2D in a large European GWA study²⁷; however, later T2D studies in Chinese²⁸, Finn²⁹ and Indian³⁰ patients failed to be replicated. In Germans, there was a modest trend of association of rs7578597 in *THADA* with T2D (p=0.055).³¹ In Chinese, *THADA* had no correlation with T2D, but it was found to be associated with 2 hour insulin levels in glucose tolerance tests.²⁸ PCOS patients with the risk allele may have greater potential to develop insulin resistance and they should be monitored for T2D.

The function of *THADA* has not been well-characterized, but some evidence suggests it might be involved in the death receptor pathway and apoptosis.³² *THADA* is extensively expressed in all tissues, and particularly high in lymphocytes, natural killer cells and monocytes. This suggests a role for *THADA* in the immune response. In the innate and/or adaptive immune response, *THADA* may participate as a cytokine in the inflammatory signaling pathway causing systemic diseases like PCOS.

*DENND1A*, the other associated 9q33.3 gene, encodes a DENN (differentially expressed in normal and neoplastic cells) domain that can hybridize with type-1 tumor necrosis factor receptor (*TNFR1*) as a negative regulator through a death domain-death domain interaction.³³ Serum *TNFR1* elevation has been previously reported to be associated with obese PCOS subjects; however, *TNF-α*was not increased synchronously in the same cohort of PCOS cases³⁴, suggesting other cytokines activate *TNF* receptor or negative regulators loss inhibitory function in the *TNF* system. We speculate that in PCOS patients having the causative *DENND1A* risk allele, *DENND1A* cannot repress normally, resulting in elevated *TNFR1* that further contributes to the induction of apoptosis and activation of the inflammation pathway.

In aggregate, both *THADA* and *DENND1A* seem to play roles in PCOS through a chronic inflammation pathogenesis. Recent studies have shown that some inflammatory mediators such as C-reactive protein (*CRP*), *TNF-α*and plasminogen activator inhibitor-1 (*PAI-1*) are increased in PCOS women.^{35,36} Obesity, present in 50-70% of PCOS³⁷, is also known to be associated with chronic systemic inflammation and increased inflammatory cytokines.³⁸ In addition, correlation between insulin resistance and inflammation has been demonstrated.³⁹ As noted, obesity and insulin resistance are two of the most prevalent features of PCOS. We speculate that the susceptibility genes we have identified, *THADA* and *DENNDIA*, may serve as proinflammatory cytokines that alters ovarian function and leads to PCOS.

Data from genotyping type 2 diabetes subjects, concurrently being analyzed, were incorporated into this study to determine whether the PCOS associations we identified also accounted for T2D. Indeed, insulin sensitivity is decreased 35% - 40% in women
with PCOS, a decrease in magnitude similar to that in T2D.⁴⁰ However, our study in Han Chinese failed to find these associations in T2D. PCOS and T2D are thus assumed to have independent susceptible factors for their pathogeneses, even while sharing the common features of obesity, metabolic abnormalities, insulin resistance and high risks for cardiovascular diseases.

In summary, our large GWA study showed that genetic variants in *LHCGR, THADA* and *DENND1A* were strongly associated with polycystic ovary syndrome but not with type 2 diabetes in Han Chinese.

Based on the description above, the present invention provides *THADA, LHCGR* and *DENND1A* genes with SNPs, wherein *THADA* gene with one or more SNPs of rs11891936 (A/G), rs17030684 (T/C), rs4340576 (G/A), rs12468394 (A/C), rs7567607 (A/G), rs13429458 (C/A), rs7568365(T/C), rs7582497 (G/A), rs10176241 (G/A), rs6744642 (T/C), rs12478601 (T/C), rs1038822(C/T), rs7559891 (A/G), rs1873555 (G/A), rs7596052 (A/G), rs10165527 (A/G), rs6726014 (T/A), rs2374551 (G/C), rs6731009 (C/T), rs10179648 (A/G) and rs7558302 (T/C) from NCBI database; *LHCGR* gene with SNP of rs13405728 (G/A) from NCBI database; *DENND1A* gene with one or more SNPs of rs10818854 (A/G), rs7857605 (C/T), rs2479106 (G/A), rs1778890 (C/T), rs1627536 (A/T) and rs10986105 (C/A) from NCBI database.

As used herein, (M/N) means minor allele/ major allele in the SNP.

The present invention further provides an entire set of probes according to claim 1 and its use according to claim 2.

The present invention further provides a method according to claim 3 wherein, this method mainly comprises the following steps: extracting the peripheral blood DNA from patients and hybridizing with the entire set of probes of claim 1; scanning and analyzing the results with the associated SNP allele information to predict the risk of PCOS.

### Brief Description of Drawings

**Figure 1****. Genome-wide Association Scan for PCOS.**
   Negative log₁₀*P* are shown for SNPs that passed quality control 5×10⁻⁸ (upper line) is the global significance level, while 10⁻⁶ is the threshold used in our study.
**Figure 2****. Association Signals for PCOS on 2p16.3, 2p21 and 9q33.3.**
   Details of the loci 2p16.3 (panel A), 2p21 (panel B) and 9q33.3 (panel C) show genome-wide significant association. Upper part shows single-marker association statistics (as -log₁₀P; left y axis) from the GWAS (circle and triangle) and the meta-analysis (squares). Recombination rates across each region in HapMap are shown in light blue (right y axis). Genomic context of each region are denoted at the lower section.

### Embodiments

### Subjects

All Han Chinese samples in this study were obtained from 24 reproductive and gynecology clinics by collaboration with multiple hospitals. Samples in the initial stage (744 PCOS cases and 895 controls) were recruited mainly from Shandong Province in Northern China; larger replications (series 1 and 2) were collected from 21 provinces throughout Northern, Central and Southern China in the mainland (3338 cases and 5792 controls).¹⁷ An additional 204 PCOS family trios were recruited for a transmission disequilibrium test. Clinical features of cases and controls are shown in Table 1. Further to elucidate whether PCOS associations account for type 2 diabetes in Han Chinese, all significant variants were checked in an unpublished T2D GWAS consisting of 1326 type 2 diabetes cases and 1159 controls (including 772 female cases and 821 female controls).

All affected individuals defined as PCOS cases were diagnosed according to the Revised 2003 Consensus on Diagnostic Criteria and Long-term Health Risks Related to Polycystic Ovary Syndrome ¹, i.e., any two of the following three criteria: oligo- or anovulation, clinical and/or biochemical signs of hyperandrogenism and polycystic ovary morphology. Patients with other causes of oligomenorrhea or hyperandrogenism were excluded. Clinical information was collected from the cases through a full clinical checkup by physician specialists. Additional demographic information was collected from both cases and controls through a structured questionnaire. All participants provided written informed consents. The study was approved by the Institutional Ethical Committee of each hospital and was conducted according to Declaration of Helsinki principles.

### Genotyping

Genome-wide genotyping was performed using the Affymetrix Genome-Wide Human SNP Array 6.0. Quality control filtering of the GWAS data was performed as follows: the arrays whose Contrast QC was 0.4 or greater being left out of further data analysis. Genotype data were generated using the birdseed algorithm. For sample filtering, array with generated genotypes of fewer than 95% of loci were excluded. For SNP filtering (after sample filtering), SNP with call rates < 95% in either case or control samples were removed. SNPs whose MAF (minor allele frequency) was < 1%, or deviated significantly from Hardy Weinberg Equilibrium (HWE, P ≤ 0.001) in controls were excluded. A total of 649,264 SNPs passed the quality criteria and were used for the subsequence analysis.

The following criteria were used for the selection of SNPs for validation: Strong significant SNPs from the GWAS were considered (allele-based P value ≤ 10-6). Those with unambiguous genotype scatter plots were excluded from the subsequent stage.

### Statistical Analysis

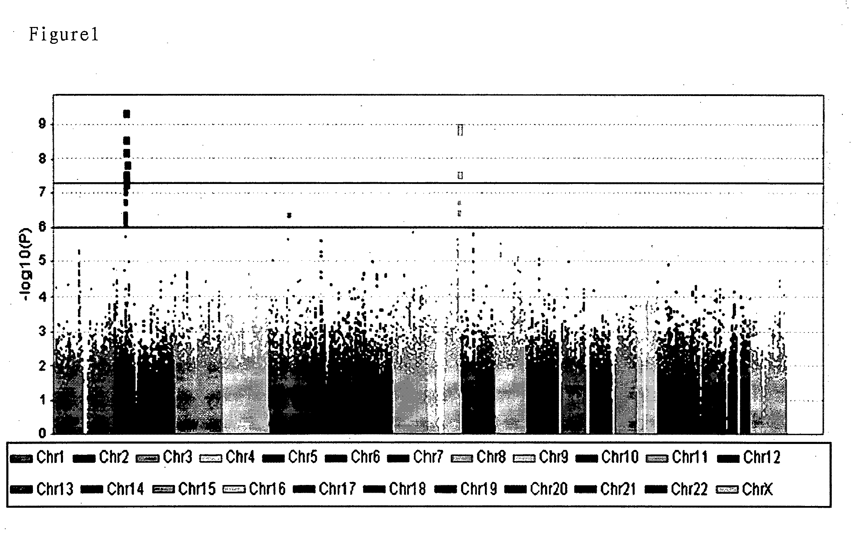

To identify possible population stratification, we performed a multi-dimensional scaling (MDS) analysis using the PLINK software package.¹⁸ Genome-wide association analysis at the single marker level and the HWE analysis in the case-control sample were performed also using PLINK. For the replication study, allelic association analysis was conducted using SHEsis.^{19,20}Haploview was used for the genome wide P value plot (Figure 1).²¹ The Q-Q plot was created using the R qq.plot function. The GWAS and replication data were combined using meta-analysis. The meta-analysis was conducted using R 'meta' package. The heterogeneity across the three stages was evaluated using Q-statistic P-value. The Mantel-Haenszel method is used to calculate the fixed effect estimate.

### Example 1: Genome Wide Association Study

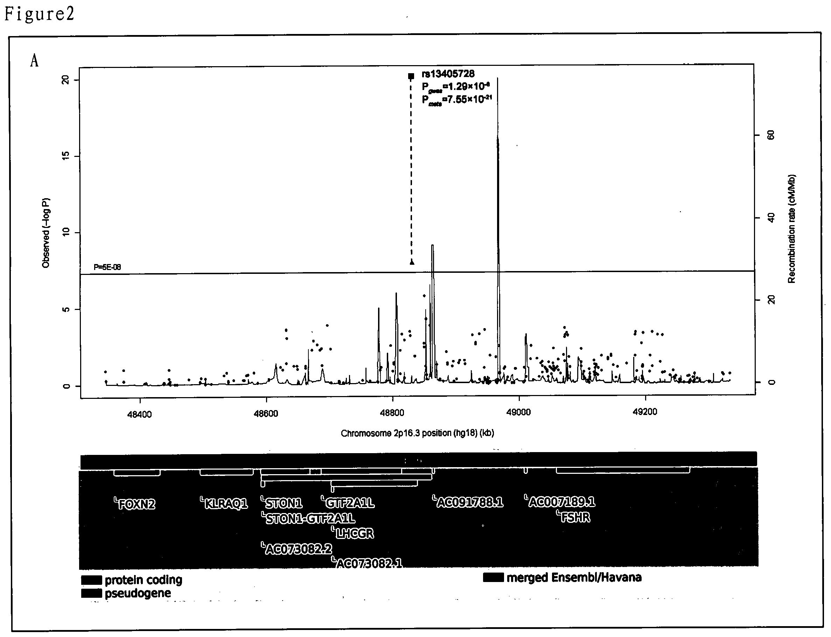

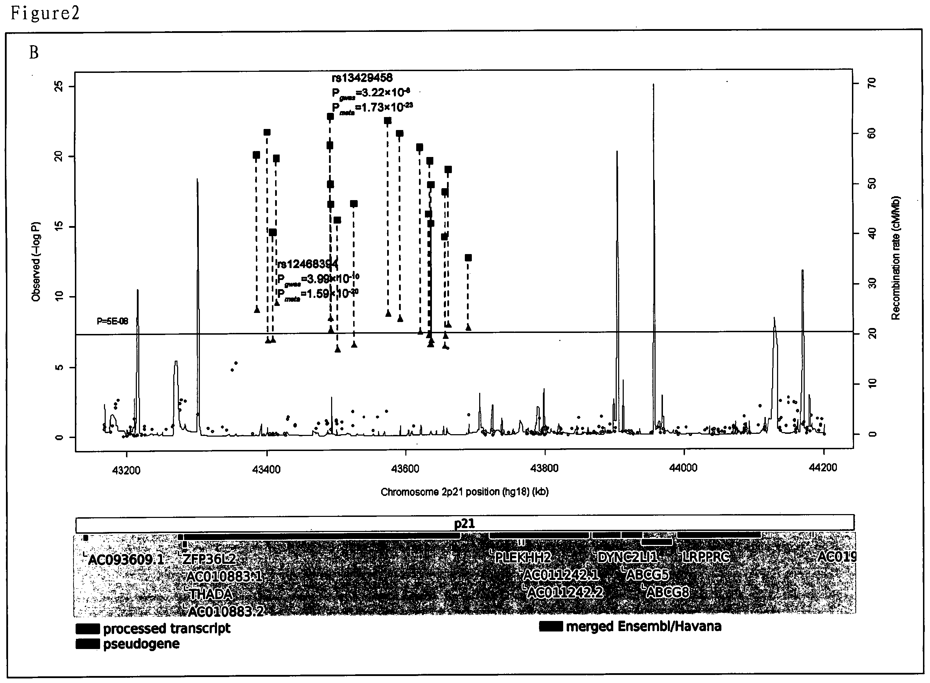

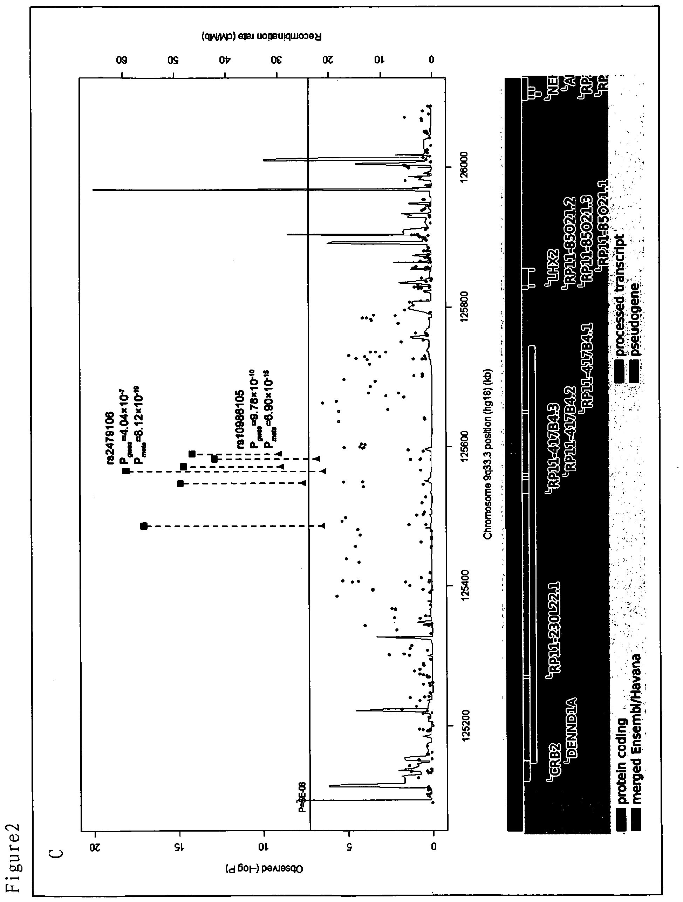

To identify genetic susceptibility loci of PCOS in Han Chinese population, we performed a GWA study by genotyping 906,600 SNPs on Affymatrix SNP 6.0 in 744 affected individuals (cases) and 895 controls. After QC filtering, a total of 649,264 SNPs remained and were subjected to statistical analysis. The genomic inflation factor (λ= 1.097) indicated a very slight inflation of the GWAS results due to population stratification, whereas MDS analysis demonstrated no obvious population substructure. An initial analysis revealed 3 distinct loci (2p16.3, 2p21 and 9q33.3, comprising 15 SNPs) showed evidence of genome-wide significant association at P < 5×10⁻⁸. The most significant SNPs for these loci were rs13405728 (allelic P value = 1.29×10⁻⁸; odds ratio, 0.61) in the gene *LHCGR* (2p16.3), rs12468394 (allelic P value = 3.99×10⁻¹⁰; odds ratio, 0.60) in the gene *THADA* (2p21), and rs10986105 (allelic P value = 9.78×10⁻¹⁰; odds ratio, 2.08) in the gene *DENND1A* (9q33.3) (Figure 1, 2).

### Example 2: Replication Studies

To validate the GWAS findings, we genotyped 28 SNPs (Table 2) - those that showed the strongest association (P < 10⁻⁶) with PCOS in the GWAS - in subjects from two replication sets: the series 1 included an independent sample set of 2840 PCOS cases and 5012 controls from Northern Han Chinese; the series 2 involved a further 498 PCOS cases and 780 controls from Central and Southern Han Chinese. In the series 1 replication, 28 of the 29 SNPs were validated. All the selected SNPs within these susceptibility loci (1 SNP of gene *LHCGR,* 21 SNPs of gene *THADA,* and 6 SNPs of gene *DENND1A*) found in GWAS were confirmed, with p values range from 1.18×10⁻⁷ to 5.55×10⁻¹⁶. Additionally, 20 remained positive in the series 2 replication study, and the most significant SNP is rs13405728 (p value = 7.93×10⁻⁷) in the gene *LHCGR* (Table 2).

### Example 3: Combined Analysis

In addition to the association tests with each of the replication sets, we also carried out a combined analysis with GWAS data using meta-analysis. The Mantel-Haenszel method is used to estimate the pooled odds ratio for all strata as a fixed effects model. After combining the GWAS and replication data, the most significant association was observed at SNP rs13429458 (2p21, Pₘₑₜₐ=1.73×10⁻²³, odds ratio=0.67, 95% CI: 0.62-0.72), rs13405728 (2p16.3, Pₘₑₜₐ=7.55×10⁻²¹, odds ratio=0.71, 95% CI: 0.67-0.77) and rs2479106 (9q33.3, Pₘₑₜₐ=8.12×10⁻¹⁹, odds ratio=1.34, 95% CI: 1.26-1.43) (Table 2, Figure 2). All selected SNPs within the three susceptibility loci showed significant association (p values range from 2.36×10⁻¹³ to 1.73×10⁻²³). The results of the combined analysis strongly support that variants in genes *LHCGR, THADA, DENND1A* were associated with susceptibility to PCOS.

### Example 4: Logistic Regression

To drop the potential effects of age and body mass index (BMI) on the results, we conducted a logistic regression analysis of disease trait using age and BMI as covariates. We used the model Y = b₀ + b₁×ADD + b₂×age+ b3.BMI+ e to fit the data, in which Y means the phenotype (1 for disease, 0 for normal), ADD means the additive effects of allele dosage (minor allele). All originally associated SNPs remained significant in the discovery and replication 1 series. In replication series 2, due to the limited number of samples, 3 SNPs were still significant after adjustment. However, the association at 2p16.3, 2p21 and 9q33.3 were not covered by the effects of age and BMI.

### Example 5: Transmission Disequilibrium Tests in PCOS Family Trios

In 204 PCOS family trios, we carried out transmission disequilibrium tests (TDT) for all 28 SNPs identified in the PCOS GWAS. Although we had limited statistical power due to the small number of trios, we still detected 6 significant SNPs (Table 3), all in the same region we previously detected. These results further support our significant findings.

### Example 6: Test PCOS Association in T2D

To elucidate whether those associations also account for T2D, we additionally investigated an unpublished T2D GWAS data set collected by our group in Han Chinese (1326 type 2 diabetes cases and 1159 controls; 772 female cases and 821 female controls). However, none of the SNPs we identified in PCOS showed nominal significance (P=0.05) in the T2D GWAS. When we looked at female samples in T2D GWAS data, only 2 SNPs (rs1778890, rs10986105) at 9q33.3 showed marginal significance, which can never pass the multiple test correction (Table 4). Obviously, the associations at 2p16.3, 2p21 and 9q33.3 with PCOS should not be byproducts of the close relationship between PCOS and T2D.

### Example 7: Predict PCOS by SNP Chip

Allele-specific oligonucleotides to the SNP sequences (Table 5 or Seq. ID No. 1-56) are printed on epoxy-coated glass microscope slide using a microarray printer. Each set was printed in duplicate so that for each SNP there were four major and minor allele oligonucleotide spots. Twelve such blocks of oligonucleotides were printed on each slide thereby allowing the testing of DNAs from twelve different people per slide.

The peripheral blood DNA from patients will be extracted and hybridized with the PCOS chip. The results with the associated SNP allele information will be scanned and analyzed to predict the risk of PCOS.

**Table 1. Characteristics of PCOS Case/Control Subjects.**

| Characteristic | Discovery Set (NHC*) | Replication1 (NHC) | Replication2 (CHC&SHC) | Family Trios |
|---|---|---|---|---|
| Cases | | | | |
| No. | 744 | 2840 | 498 | 204 |
| Age(ys) | 28.85±3.62 | 28.28±3.99 | 27.00±4.00 | 27.04±3.71 |
| BMI | 24.55±3.99 | 24.76±4.74 | 23.28±4.13 | 25.17±4.57 |
| T (ng/dl) | 81.11±21.03 | 61.17±27.27 | 61.56±34.51 | 70.46±24.28 |
| HOMA-IR | 2.27±1.72 | 2.64±2.13 | 3.84±3.56 | 2.23±1.55 |
| Controls | | | | |
| No. | 895 | 5012 | 780 | - |
| Age(ys) | 30.68±4.68 | 31.38±7.73 | 31.31±4.07 | - |

| | | | | |
|---|---|---|---|---|
| *NHC, CHC and SHC denote Northern Han Chinese, Central Han Chinese and Southern Han Chinese respectively; BMI: body mass index; T: testosterone; HOMA-IR: homeostasis model assessment-insulin resistance. | | | | |

**Table 2. GWAS, Case Control Replication Study and Meta-analysis Results for the Most Significant SNPs**

| | dbSNP RSID | SNP Chr | Physical Position | Cytoband | Transcript ID | Gene Symbol | Allele A | Allele B | SNP Strand | Relationship |
|---|---|---|---|---|---|---|---|---|---|---|
| 1 | rs11891936 | 2 | 43385806 | p21 | NM_001083953 | THADA | A | G | - | intron |
| 2 | rs17030684 | 2 | 43401632 | p21 | NM_001083953 | THADA | C | T | - | intron |
| 3 | rs4340576 | 2 | 43409010 | p21 | NM_001083953 | THADA | A | G | - | intron |
| 4 | rs12468394 | 2 | 43414665 | p21 | NM_001083953 | THADA | A | C | + | intron |
| 5 | rs7567607 | 2 | 43491689 | p21 | NM_001083953 | THADA | A | G | - | intron |
| 6 | rs13429458 | 2 | 43492342 | p21 | NM_001083953 | THADA | A | C | + | intron |
| 7 | rs7568365 | 2 | 43492359 | p21 | NM_001083953 | THADA | C | T | + | intron |
| 8 | rs7582497 | 2 | 43492451 | p21 | NM_001083953 | THADA | A | G | - | intron |
| 9 | rs10176241 | 2 | 43501680 | p21 | NM_001083953 | THADA | A | G | + | intron |
| 10 | rs6744642 | 2 | 43525408 | p21 | NM_001083953 | THADA | C | T | + | intron |
| 11 | rs12478601 | 2 | 43575012 | p21 | NM_001083953 | THADA | C | T | + | intron |
| 12 | rs1038822 | 2 | 43591677 | p21 | NM_001083953 | THADA | C | T | + | intron |
| 13 | rs7559891 | 2 | 43620543 | p21 | NM_001083953 | THADA | A | G | + | intron |
| 14 | rs1873555 | 2 | 43633169 | p21 | NM_001083953 | THADA | A | G | - | intron |
| 15 | rs7596052 | 2 | 43635330 | p21 | NM_001083953 | THADA | A | G | + | intron |
| 16 | rs10165527 | 2 | 43636372 | p21 | NM_001083953 | THADA | A | G | - | intron |
| 17 | rs6726014 | 2 | 43636798 | p21 | NM_001083953 | THADA | A | T | + | intron |
| 18 | rs2374551 | 2 | 43655884 | p21 | NM_001083953 | THADA | C | G | + | intron |
| 19 | rs6731009 | 2 | 43656932 | p21 | NM_001083953 | THADA | C | T | + | intron |
| 20 | rs10179648 | 2 | 43661569 | p21 | NM_001083953 | THADA | A | G | - | intron |
| 21 | rs7558302 | 2 | 43690191 | p21 | NM_022065 | THADA | C | T | + | upstream |
| 22 | rs13405728 | 2 | 48831663 | p16.3 | NM_000233 | LHCGR | A | G | + | intron |
| 23 | rs10818854 | 9 | 125486599 | q33.3 | NM_024820 | DENND1A | A | G | + | intron |
| 24 | rs7857605 | 9 | 125547434 | q33.3 | NM_024820 | DENND1A | C | T | + | intron |
| 25 | rs2479106 | 9 | 125565033 | q33.3 | NM_024820 | DENND1A | A | G | + | intron |
| 26 | rs1778890 | 9 | 125571576 | q33.3 | NM_024820 | DENND1A | C | T | + | intron |
| 27 | rs1627536 | 9 | 125582525 | q33.3 | NM_024820 | DENND1A | A | T | - | intron |
| 28 | rs10986105 | 9 | 125589776 | q33.3 | NM_024820 | DENND1A | A | C | - | intron |

**Table 3. Family-base Association Test Results for the Replication SNPs.**

| SNP | CHR | BP | Allele^{a} | T^{b} | U | OR | CHI-2 | P |
|---|---|---|---|---|---|---|---|---|
| rs17030684 | 2 | 43401632 | T/C | 42 | 82 | 0.5122 | 12.9 | 0.000328 |
| rs4340576 | 2 | 43409010 | C/T | 46 | 44 | 1.045 | 0.04444 | 0.833 |
| rs12468394 | 2 | 43414665 | A/C | 31 | 47 | 0.6596 | 3.282 | 0.07004 |
| rs7567607 | 2 | 43491689 | T/C | 53 | 63 | 0.8413 | 0.8621 | 0.3532 |
| rs13429458 | 2 | 43492342 | G/T | 31 | 63 | 0.4921 | 10.89 | 0.000965 |
| rs7568365 | 2 | 43492359 | T/C | 53 | 63 | 0.8413 | 0.8621 | 0.3532 |
| rs7582497 | 2 | 43492451 | C/T | 32 | 45 | 0.7111 | 2.195 | 0.1385 |
| rs10176241 | 2 | 43501680 | G/A | 37 | 63 | 0.5873 | 6.76 | 0.009322 |
| rs6744642 | 2 | 43525408 | T/C | 42 | 47 | 0.8936 | 0.2809 | 0.5961 |
| rs12478601 | 2 | 43575012 | A/G | 53 | 61 | 0.8689 | 0.5614 | 0.4537 |
| rs1038822 | 2 | 43591677 | G/A | 55 | 77 | 0.7143 | 3.667 | 0.05551 |
| rs7559891 | 2 | 43620543 | T/C | 46 | 52 | 0.8846 | 0.3673 | 0.5445 |
| rs1873555 | 2 | 43633169 | C/T | 30 | 46 | 0.6522 | 3.368 | 0.06646 |
| rs7596052 | 2 | 43635330 | A/G | 38 | 59 | 0.6441 | 4.546 | 0.03299 |
| rs10165527 | 2 | 43636372 | T/C | 26 | 37 | 0.7027 | 1.921 | 0.1658 |
| rs6726014 | 2 | 43636798 | T/A | 40 | 63 | 0.6349 | 5.136 | 0.02344 |
| rs2374551 | 2 | 43655884 | G/C | 38 | 66 | 0.5758 | 7.538 | 0.00604 |
| rs6731009 | 2 | 43656932 | C/T | 29 | 37 | 0.7838 | 0.9697 | 0.3248 |
| rs10179648 | 2 | 43661569 | T/C | 55 | 62 | 0.8871 | 0.4188 | 0.5175 |
| rs7558302 | 2 | 43690191 | T/C | 57 | 75 | 0.76 | 2.455 | 0.1172 |
| rs13405728 | 2 | 48831663 | G/A | 34 | 52 | 0.6538 | 3.767 | 0.05226 |
| rs10514258 | 5 | 82907513 | G/A | 21 | 21 | 1 | 0 | 1 |
| rs10818854 | 9 | 125486599 | A/G | 26 | 21 | 1.238 | 0.5319 | 0.4658 |
| rs7857605 | 9 | 125547434 | G/A | 32 | 32 | 1 | 0 | 1 |
| rs2479106 | 9 | 125565033 | G/A | 55 | 43 | 1.279 | 1.469 | 0.2254 |
| rs1778890 | 9 | 125571576 | C/T | 45 | 34 | 1.324 | 1.532 | 0.2159 |
| rs1627536 | 9 | 125582525 | T/A | 53 | 51 | 1.039 | 0.03846 | 0.8445 |
| rs10986105 | 9 | 125589776 | C/A | 38 | 32 | 1.188 | 0.5143 | 0.4733 |

| | | | | | | | | |
|---|---|---|---|---|---|---|---|---|
| ^{a}Minor allele/major allele. ^{b}Transmitted minor allele count. | | | | | | | | |

**Table 4. PCOS Associated SNPs in Diabetes Cases and Controls.**

| SNP | CHR | BP | Allele^{a} | Diabetes | | | | | | | |
|---|---|---|---|---|---|---|---|---|---|---|---|
| | | | | 1326 cases, 1159 controls | | | | 772 female cases, 821 female controls | | | |
| | | | | MAF | | OR | P | MAF | | OR | P |
| | | | | Case | Control | | | Case | Control | | |
| rs11891936 | 2 | 43385806 | A/G | 0.221 | 0.208 | 1.08 | 0.28 | 0.207 | 0.220 | 0.93 | 0.39 |
| rs17030684 | 2 | 43401632 | T/C | 0.224 | 0.206 | 1.12 | 0.12 | 0.211 | 0.219 | 1.05 | 0.58 |
| rs4340576 | 2 | 43409010 | G/A | 0.297 | 0.291 | 1.03 | 0.61 | 0.283 | 0.308 | 1.12 | 0.13 |
| rs12468394 | 2 | 43414665 | A/C | 0.268 | 0.260 | 1.04 | 0.52 | 0.254 | 0.275 | 0.90 | 0.18 |
| rs7567607 | 2 | 43491689 | A/G | 0.304 | 0.288 | 1.08 | 0.24 | 0.294 | 0.305 | 0.95 | 0.48 |
| rs13429458 | 2 | 43492342 | C/A | 0.190 | 0.181 | 1.07 | 0.39 | 0.182 | 0.195 | 0.92 | 0.37 |
| rs7568365 | 2 | 43492359 | T/C | 0.298 | 0.286 | 1.06 | 0.35 | 0.284 | 0.299 | 0.93 | 0.38 |
| rs7582497 | 2 | 43492451 | G/A | 0.302 | 0.287 | 1.07 | 0.27 | 0.289 | 0.303 | 0.94 | 0.40 |
| rs10176241 | 2 | 43501680 | G/A | 0.301 | 0.289 | 1.06 | 0.36 | 0.291 | 0.304 | 0.94 | 0.43 |
| rs6744642 | 2 | 43525408 | T/C | 0.213 | 0.199 | 1.09 | 0.22 | 0.200 | 0.211 | 0.93 | 0.45 |
| rs12478601 | 2 | 43575012 | T/C | 0.292 | 0.282 | 1.05 | 0.45 | 0.282 | 0.298 | 0.92 | 0.30 |
| rs1038822 | 2 | 43591677 | C/T | 0.311 | 0.297 | 1.07 | 0.31 | 0.304 | 0.313 | 0.96 | 0.60 |
| rs7559891 | 2 | 43620543 | A/G | 0.298 | 0.288 | 1.05 | 0.43 | 0.287 | 0.303 | 0.93 | 0.33 |
| rs1873555 | 2 | 43633169 | G/A | 0.243 | 0.230 | 1.07 | 0.32 | 0.229 | 0.243 | 0.93 | 0.36 |
| rs7596052 | 2 | 43635330 | A/G | 0.237 | 0.227 | 1.06 | 0.43 | 0.224 | 0.238 | 0.92 | 0.34 |
| rs10165527 | 2 | 43636372 | A/G | 0.239 | 0.230 | 1.05 | 0.45 | 0.224 | 0.243 | 0.90 | 0.21 |
| rs6726014 | 2 | 43636798 | T/A | 0.236 | 0.228 | 1.05 | 0.46 | 0.224 | 0.239 | 0.92 | 0.31 |
| rs2374551 | 2 | 43655884 | G/C | 0.238 | 0.228 | 1.05 | 0.44 | 0.223 | 0.241 | 0.91 | 0.25 |
| rs6731009 | 2 | 43656932 | C/T | 0.247 | 0.237 | 1.06 | 0.41 | 0.233 | 0.250 | 0.91 | 0.27 |
| rs10179648 | 2 | 43661569 | A/G | 0.302 | 0.288 | 1.07 | 0.29 | 0.293 | 0.302 | 0.96 | 0.56 |
| rs7558302 | 2 | 43690191 | T/C | 0.316 | 0.312 | 1.02 | 0.76 | 0.31 | 0.323 | 0.94 | 0.42 |
| rs13405728 | 2 | 48831663 | G/A | 0.239 | 0.26 | 0.89 | 0.09 | 0.244 | 0.269 | 0.88 | 0.12 |
| rs10818854 | 9 | 125486599 | A/G | 0.081 | 0.080 | 1.02 | 0.87 | 0.086 | 0.072 | 1.22 | 0.13 |
| rs7857605 | 9 | 125547434 | C/T | 0.083 | 0.078 | 1.07 | 0.52 | 0.088 | 0.07 | 1.29 | 0.06 |
| rs2479106 | 9 | 125565033 | G/A | 0.228 | 0.218 | 1.06 | 0.39 | 0.235 | 0.214 | 1.13 | 0.16 |
| rs1778890 | 9 | 125571576 | C/T | 0.155 | 0.141 | 1.12 | 0.15 | 0.163 | 0.137 | 1.22 | 0.04 |
| rs1627536 | 9 | 125582525 | A/T | 0.226 | 0.218 | 1.05 | 0.49 | 0.233 | 0.214 | 1.12 | 0.20 |
| rs10986105 | 9 | 125589776 | C/A | 0.088 | 0.080 | 1.12 | 0.28 | 0.096 | 0.072 | 1.37 | 0.01 |

| | | | | | | | | | | | |
|---|---|---|---|---|---|---|---|---|---|---|---|
| ^{a}Minor allele/major allele. | | | | | | | | | | | |

**Table 5. SNPs Probes for PCOS Chip.**

| | dbSNP RSID | Strand | Probe sequence |
|---|---|---|---|
| 1 | rs11891936 | + | |
| 2 | rs17030684 | + | |
| 3 | rs4340576 | + | |
| 4 | rs12468394 | + | |
| 5 | rs7567607 | + | |
| 6 | rs13429458 | + | |
| 7 | rs7568365 | + | |
| 8 | rs7582497 | + | |
| 9 | rs10176241 | + | |
| 10 | rs6744642 | + | |
| 11 | rs12478601 | + | |
| 12 | rs1038822 | + | |
| 13 | rs7559891 | + | |
| 14 | rs1873555 | + | |
| 15 | rs7596052 | + | |
| 16 | rs10165527 | + | |
| 17 | rs6726014 | + | |
| 18 | rs2374551 | + | |
| 19 | rs6731009 | + | |
| 20 | rs10179648 | + | |
| 21 | rs7558302 | + | |
| 22 | rs13405728 | + | |
| 23 | rs10818854 | + | |
| 24 | rs7857605 | + | |
| 25 | rs2479106 | + | |
| 26 | rs1778890 | + | |
| 27 | rs1627536 | + | |
| 28 | rs10986105 | + | |

### REFERENCES

1. Rotterdam ESHRE/ASRM-Sponsored PCOS Consensus Workshop Group: Revised 2003 consensus on diagnostic criteria and longterm health risks related to polycystic ovary syndrome. Fertil Steril 2004; 81: 19-25.
2. Goodarzi MO, Azziz R. Diagnosis, epidemiology, and genetics of the polycystic ovary syndrome. Best Prac Res Clin Endocrinol Metab 2006; 20: 193-200.
3. Ehrmann DA, Barnes RB, Rosenfield RL, Cavaghan MK, Imperial J. Prevalence of impaired glucose tolerance and diabetes in women with polycystic ovary syndrome. Diabetes Care 1999; 22: 141-6.
4. Carmina E. Cardiovascular risk and events in polycystic ovary syndrome. Climacteric 2009; 12 Suppl 1:22-5.
5. Kandaraki E, Christakou C, Diamanti-Kandarakis E. Metabolic syndrome and polycystic ovary syndrome... and vice versa. Arq Bras Endocrinol Metabol 2009; 53:227-37.
6. Wild S, Pierpoint T, Jacobs H, McKeigue P. Long-term consequences of polycystic ovary syndrome: results of a 31 year follow-up study. Hum Fertil(Camb) 2000; 3:101-5.
7. Legro RS, Castracane VD, Kauffman RP. Detecting insulin resistance in polycystic ovary syndrome: purposes and pitfalls. Obstet Gynecol Surv 2004; 59:141-54.
8. Espinós-Gómez JJ, Corcoy R, Calaf J. Prevalence and predictors of abnormal glucose metabolism in Mediterranean women with polycystic ovary syndrome. Gynecol Endocrinol 2009; 25:199-204.
9. Kulshreshtha B, Ganie MA, Praveen EP, et al. Insulin response to oral glucose in healthy, lean young women and patients with polycystic ovary syndrome. Gynecol Endocrinol 2008; 24: 637-43.
10. Shi Y, Guo M, Yan J, et al. Analysis of clinical characteristics in large-scale Chinese women with polycystic ovary syndrome. Neuro Endocrinol Lett 2007; 28: 807-10.
11. Sudo S, Kudo M, Wada S, Sato O, Hsueh AJ, Fujimoto S. Genetic and functional analyses of polymorphisms in the human FSH receptor gene. Mol Hum Reprod 2002; 8:893-9.
12. Gaasenbeek M, Powell BL, Sovio U, et al. Large-scale analysis of the relationship between CYP11A promoter variation, polycystic ovarian syndrome, and serum testosterone. J Clin Endocrinol Metab 2004; 89:2408-13.
13. Wang Y, Wu X, Cao Y, Yi L, Chen J. A microsatellite polymorphism (tttta)n in the promoter of the CYP1 la gene in Chinese women with polycystic ovary syndrome. Fertil Steril 2006; 86: 223-6.
14. Chen ZJ, Shi YH, Zhao YR, et al. Correlation between single nucleotide polymorphism of insulin receptor gene with polycystic ovary syndrome. Zhonghua Fu Chan Ke Za Zhi 2004; 39: 582-5.
15. Villuendas G, San Millán JL, Sancho J, Escobar-Morreale HF. The -597 G-->A and-174 G-->C polymorphisms in the promoter of the IL-6 gene are associated with hyperandrogenism. J Clin Endocrinol Metab 2002; 87: 1134-41.
16. Simoni M, Tempfer CB, Destenaves B, Fauser BC. Functional genetic polymorphisms and female reproductive disorders: Part I: Polycystic ovary syndrome and ovarian response. Hum Reprod Update 2008; 14: 459-84
17. Xu S, Yin X, Li S, et al. Genomic dissection of population substructure of Han Chinese and its implication in association studies. Am J Hum Genet 2009; 85: 762-74.
18. Purcell S, Neale B, Todd-Brown K, et al. PLINK: a tool set for whole-genome association and population-based linkage analyses. Am J Hum Genet 2007; 81: 559-75.
19. Shi YY, He L. SHEsis, a powerful software platform for analyses of linkage disequilibrium, haplotype construction, and genetic association at polymorphism loci. Cell research 2005; 15, 97-8.
20. Li Z, Zhang Z, He Z, et al. A partition-ligation-combination-subdivision EM algorithm for haplotype inference with multiallelic markers: update of the SHEsis (http://analysis.bio-x.cn). Cell Res 2009; 19: 519-23.
21. Barrett, J.C., Fry, B., Maller, J. and Daly, M.J. Haploview: analysis and visualization of LD and haplotype maps. Bioinformatics 2005; 21, 263.
22. Segaloff DL. Chapter 4 Diseases Associated with Mutations of the Human Lutropin Receptor. Prog Mol Biol Transl Sci 2009; 89C: 97-114.
23. Latronico AC, Chai Y, Arnhold IJP, Liu X, Mendonca BB, Segaloff DL. A homozygous microdeletion in helix seven of the luteinizing hormone receptor associated with familial testicular and ovarian resistance is due to both decreased cell surface expression and impaired Gs activation by the cell surface receptor. Mol Endocrinol 1998; 12: 442-50.
24. Toledo SPA, Brunner HG, Kraaij R,et al. An inactivating mutation of the luteinizing hormone receptor causes amenorrhea in a 46, XX female. J Clin Endocrinol Metab 1996; 81: 3850-4.
25. Latronico AC, Lins TS, Brito VN, Arnhold IJ, Mendonca BB. The effect of distinct activating mutations of the luteinizing hormone receptor gene on the pituitary-gonadal axis in both sexes. Clin Endocrinol (Oxf) 2000; 53: 609-13.
26. Drieschner N, Beige G, Rippe V, Meiboom M, Loeschke S, Bullerdiek J. Evidence for a 3p25 breakpoint hot spot region in thyroid tumors of follicular origin. Thyroid 2006; 16: 1091-6.
27. Zeggini E, Scott LJ, Saxena R,et al. Meta-analysis of genome-wide association data and large-scale replication identifies additional susceptibility loci for type 2 diabetes. Nat Genet 2008; 40: 638-45.
28. Hu C, Zhang R, Wang C, et al. PPARG, KCNJ11, CDKAL1, CDKN2A-CDKN2B, IDE-KIF11-HHEX, IGF2BP2 and SLC30A8 are associated with type 2 diabetes in a Chinese population. PLoS One 2009; 4: e7643.
29. Stancáková A, Kuulasmaa T, Paananen J, et al. Association of 18 confirmed susceptibility loci for type 2 diabetes with indices of insulin release, proinsulin conversion, and insulin sensitivity in 5,327 nondiabetic Finnish men. Diabetes 2009; 58: 2129-36.
30. Sanghera DK, Been L, Ortega L, et al. Testing the association of novel meta-analysis-derived diabetes risk genes with type II diabetes and related metabolic traits in Asian Indian Sikhs. J Hum Genet 2009; 54: 162-8.
31. Schleinitz D, Tönjes A, Böttcher Y, et al. Lack of significanteffects of the type 2 diabetes susceptibility loci JAZF1, CDC123/CAMK1D, NOTCH2, ADAMTS9, THADA, and TSPAN8/LGR5 on diabetes and quantitative metabolic traits. Horm Metab Res 2010; 42: 14-22.
32. Drieschner N, Kerschling S, Soller JT N, et al. A domain of the thyroid adenoma associated gene (THADA) conserved in vertebrates becomes destroyed by chromosomal rearrangements observed in thyroid adenomas Gene 2007; 403: 110-7.
33. Del Villar K, Miller CA. Down-regulation of DENN/MADD, a TNF receptor binding protein, correlates with neuronal cell death in Alzheimer's disease brain and hippocampal neurons. Proc Natl Acad Sci USA 2004; 101: 4210-5.
34. Olszanecka-Glinianowicz M, Banas M, Zahorska-Markiewicz B, et al. Is the polycystic ovary syndrome associated with chronic inflammation per se? Eur J Obstet Gynecol Reprod Biol 2007; 133: 197-202.
35. Benson S, Janssen OE, Hahn S, et al. Obesity, depression, and chronic low-grade inflammation in women with polycystic ovary syndrome. Brain Behav Immun 2008; 22: 177-84.
36. González F, Rote NS, Minium J, Kirwan JP. Evidence of proatherogenic inflammation in polycystic ovary syndrome. Metabolism. 2009; 58: 954-62.
37. Ehrmann DA, Barnes RB, Rosenfield RL, Cavaghan MK, Imperial J. Prevalence of impaired glucose tolerance and diabetes in women with polycystic ovary syndrome. Diabetes Care 1999; 22: 141-6.
38. Iyer A, Fairlie DP, Prins JB, Hammock BD, Brown L. Inflammatory lipid mediators in adipocyte function and obesity. Nat Rev Endocrinol 2010; 6: 71-82.
39. Bloomgarden ZT. Inflammation and insulin resistance. Diabetes Care 2003; 26: 1922-6.
40. O'Driscoll JB, Mamtora H, Higginson J, Pollock A, Kane J, Anderson DC. A prospective study of the prevalence of clear-cut endocrine disorders and polycystic ovaries in 350 patients presenting with hirsutism or androgenic alopecia. Clin Endocrinol (Oxf) 1994; 41:231-6.

## Claims

1. An entire set of probes with SNPs used for predicting the risk of PCOS, wherein the nucleotide sequences of the probes are shown as Seq ID No. 1-56.

2. The use of the entire set of probes of Claim 1 in predicting the risk of PCOS.

3. A method for predicting the risk of PCOS, wherein the method uses the entire set of probes of Claim 1.

4. The method of claim 3, wherein the method mainly comprises the following steps: extracting the peripheral blood DNA from patients and hybridizing with the entire set of probes of Claim 1; scanning and analyzing the results with the associated SNP allele information to predict the risk of PCOS.

## Patentansprüche

1. Vollständiger Satz von Proben mit SNPs, die zur Prognose der Gefahr des polyzystischen Ovarialsyndroms verwendet werden, wobei die Nukleotidsequenzen der Proben durch Seq. ID Nr. 1-56 dargestellt werden.

2. Verwendung des vollständigen Satzes von Proben nach Anspruch 1 bei der Prognose der Gefahr des polyzystischen Ovarialsyndroms.

3. Verfahren zur Prognose der Gefahr des polyzystischen Ovarialsyndroms, wobei das Verfahren den vollständigen Satz von Proben nach Anspruch 1 verwendet.

4. Verfahren nach Anspruch 3, wobei das Verfahren in erster Linie die folgenden Schritte umfasst:
Extrahieren der peripheren Blut-DNA der Patienten und Hybridisieren mit dem vollständigen Satz von Proben nach Anspruch 1; Scannen und Analysieren der Ergebnisse mit der assoziierten SNP-Allel-Information zur Prognose der Gefahr des polyzystischen Ovarialsyndroms.

## Revendications

1. Ensemble complet de sondes avec des SNP utilisé pour prédire le risque de PCOS, dans lequel les séquences nucléotidiques des sondes sont représentées en tant que Seq ID N° 1 à 56.

2. Utilisation de l'ensemble complet de sondes selon la revendication 1 dans la prédiction du risque de PCOS.

3. Procédé pour prédire le risque de PCOS, dans lequel le procédé utilise l'ensemble complet de sondes selon la revendication 1.

4. Procédé selon la revendication 3, dans lequel le procédé comprend principalement les étapes suivantes : extraction de l'ADN du sang périphérique de patients et hybridation avec l'ensemble complet de sondes selon la revendication 1 ; balayage et analyse des résultats avec les informations sur l'allèle SNP associées pour prédire le risque de PCOS.
